# EUROPEAN PATENT SPECIFICATION

(11) **EP 2 197 415 B1**
(45) Date of publication and mention of the grant of the patent: **05.03.2014**
(21) Application number: 08808054.4
(22) Date of filing: 18.09.2008
(51) Int. Cl.: A61K 9/46, A61K 9/00, A61K 31/575

(54) **COMPOSITIONS AND MEANS FOR TREATING UTERINE LEIOMYOMATA, LEIOMYOMA, MYOMA, UTERINE FIBROIDS, ENDROMETRIOSIS, ADENOMYOSIS AND RELATED DISORDERS BY MIFEPRISTONE**
ZUSAMMENSETZUNGEN UND MITTEL ZUR BEHANDLUNG VON GEBÄRMUTTER-LEIOMYOMATA, LEIOMYOMEN, MYOMEN, GEBÄRMUTTERFIBROIDEN, ENDROMETRIOSE, ADENOMYOSE UND VERWANDTEN ERKRANKUNGEN MIT MIFEPRISTON
COMPOSITIONS ET MOYENS PERMETTANT DE TRAITER LA LÉIMOYOMATOSE UTÉRINE, LE LÉIOMYOME, LES FIBROÏDES UTÉRINS, L'ENDOMÉTRIOSE, L'ADÉNOMYOSE ET DES TROUBLES APPARENTÉS PAR MIFÉPRISTONE

(30) Priority: 20.09.2007 US 960205 P
(43) Date of publication of application: 23.06.2010
(73) Proprietor: LAPIDOT MEDICAL IMPORT AND MARKETING LTD, Israel 38900 (IL)
(72) Inventor: KATZ, Daniel, 20179 Misgav (IL)
(74) Representative: Lecomte, Didier
(86) International application number: PCT/IL2008/001253
(87) International publication number: WO 2009/037704

(56) References cited:
- WO-A-03/017974
- CN-A- 1 846 703
- US-A1- 2005 197 651
- US-A1- 2007 213 306

## Description

### FIELD OF THE INVENTION

This invention is directed towards treating uterine conditions in women, more specifically the invention relates to a vaginal tablet comprising mifepristone compositions. The invention also pertains to a method for treating uterine disorders, especially leiomyomata, leiomyoma, myoma, uterine fibroids, endometriosis, adenomyosis and related disorders by mifepristone.

### BACKGROUND OF THE INVENTION

Uterine leiomyomata are common, benign pelvic tumors occurring in up to 35% of women aged more than 35 years and account for up to 40% of all hysterectomies. Uterine fibroids are benign tumors of the uterus made up of smooth muscle and the extracellular matrix proteins Collagen and Elastin. They are exceptionally common; the cumulative incidence of a diagnosis of fibroids in women aged 25 to 45 is approximately 30 percent.

Uterine fibroids can cause abnormal uterine bleeding, dysmenorrhea, lower back pain and non-cyclic pelvic pain. They also can contribute to symptoms related to an enlarging, pelvic mass (e.g., urinary frequency or constipation).

Uterine fibroids are also associated with an increased risk of complications of pregnancy, and with infertility, although it is unclear whether this association is causative. Symptoms associated with uterine fibroids can have a significant impact on quality of life, with scores on standard measures that are comparable to those for other major chronic diseases.

Present treatments for uterine fibroids include:

### Invasive therapies:

- Uterine artery embolization - non-surgical treatment that blocks the blood vessels that "feed" the uterine fibroid, causing it to shrink.
- Coagulation using cautery or laser.
- Myomectomy - removal of the uterine fibroid in a surgical procedure (high probability of complications).
- Laparoscopic myomectomy.
- Hysterectomy - removal of the entire uterus in a surgical procedure (high probability of complications).
- MR-guided focused ultrasound- obliterates tumors by focusing high-intensity ultrasound beams on the growths, raising the temperature enough to destroy them. The treatment is guided by magnetic resonance (MR) images.

### Medical therapies:

- Nonsteroidal anti-inflammatory drugs (NSAIDs).
- Oral contraceptive pills (OCPs).
- Progestational agents.
- Other oral agents identified in the literature search (e.g., mifepristone, tibolone, herbal preparations).
- Gonadotropin-releasing hormone (GnRH) agonists (both as primary therapy and as an adjunct to myomectomy or hysterectomy) - drugs that block estrogen production, depriving uterine fibroids of estrogen and causing them to shrink.

### No intervention ("watchful waiting").

Until recently, nonsurgical treatment options for symptomatic leiomyomata were limited. Treatment with gonadotropin-releasing hormone (GnRH) agonists results in a decrease in leiomyoma size of approximately 36% after 12 weeks and a significant decrease in associated symptoms. Within 6 months of treatment completion, however, the uterus returns to pretreatment size and symptoms recur. Although the side effects of hypoestrogenism limit treatment length, it has been shown to have clinically beneficial outcomes in short-term, presurgical settings. Uterine artery embolization has been shown to decrease leiomyoma size by 35-69%, improve menorrhagia, and reduce pain, but limited experience has shown potential complications. No randomized, controlled trials have been conducted, and long-term efficacy has not been reported.

Studies have suggested that leiomyomata growth is steroid-dependent and that mitotic activity in leiomyomata is greatest in the luteal phase. Recent studies have provided further biochemical, histological, and clinical evidence that progesterone has a critical role in leiomyoma growth. Recently, mifepristone, a progesterone receptor modulator with primarily antagonistic properties, has been shown to decrease leiomyoma size.

The availability of a safe and effective nonsurgical treatment of symptomatic uterine fibroids has a considerable clinical, economical and public health importance.

Patent application WO9808471 of MOO-YOUNG et al. teaches the vaginal application of Mifepristone as a method of contraception. Mifepristone is administered via a device such as a patch, vaginal ring, vaginal or uterine implant over a multiday period. Patent application US5468741 to Yen teaches the use of low levels mifepristone to treat leimyomata, orally administered. The 741' patent discloses vaginally administered mifepristone by intravaginal and intrauterine implants which slowly release mifepristone. Implants of this type have several drawbacks, they are moderately invasive, often inconvenient to use, uncomfortable, potential sources of chronic infections and may promote an inflammatory reaction.

Patent application W003017974A1 of Pantarhei Bioscience B.V. relates to the treatment of benign gynecological disorders by intravaginal administration of a drug delivery vehicle comprising anti estrogen. The anti-estrogen can be administered in a pharmaceutical kit, optionally, additionally comprising mifepristone. The mifepeistone is administered via a slow release intravaginal delivery vehicle, releasing low amounts of the drug over a long period of time, without intermission. The aforementioned publication specifically teaches the treatment of uterine fibroids with the combination of raloxifene and mifepristone. It is declared in this publication that the combination of raloxifen and mifepristone is more efficacious in reduction of fibroid size than the results obtained by using raloxifene alone. However, the therapeutic efficacy of vaginally administered mifepristone, formulated in other delivery forms i.e. immediate release form is still to be explored.

It would be beneficial to the patient to combine the effectiveness of mifepristone treatment of leimyomata with the ability to administer it vaginally, avoiding the inconveniences and problems associated with oral administration such as the 'burst" effect, patient compliance and relatively high oral doses in order to achieve an effective concentration of the drug at the target tissue. There thus remains a long felt need to provide means and method of treating uterine leiomyomata, leiomyoma, myoma, uterine fibroids and related disorders with a vaginally introduced therapeutic composition which is not associated with an implant.

### SUMMARY OF THE INVENTION

It is one object of the invention to provide a vaginally administrable tablet useful for treating leiomyomata, leiomyoma, myoma, uterine fibroids, endometriosis, adenomyosis and other uterine disorders. The tablet comprises mifepristone, at least one non-effervescent excipient or diluent, and at least one effervescent excipient,
wherein said tablet is formulated in an immediate release form.

It is another object of the invention to disclose the vaginally administrable tablet wherein the tablet has a disintegration time of less than about 15 minutes in the vagina.

It is another object of the invention to disclose the vaginally administrable tablet wherein the tablet has disintegration time of between about 15 minutes and about 60 minutes in the vagina.

It is another object of the invention to disclose the vaginally administrable tablet wherein the tablet has a disintegration time of less than about 3 minutes in water at room temperature.

It is another object of the invention to disclose the vaginally administrable tablet wherein the tablet has disintegration time of between about 3 minutes and about 15 minutes in water at room temperature.

It is another object of the invention to disclose the vaginally administrable tablet wherein the tablet comprises between about 0.1 to about 50 mg of mifepristone and between about 5 to about 12 wt.% effervescent excipient.

It is another object of the invention to disclose the vaginally administrable tablet wherein the-tablet comprises between about 5 to about 20 mg of mifepristone.

It is another object of the invention to disclose the vaginally administrable tablet wherein the tablet comprises between about 6 to 8 wt. % effervescent excipient.

It is another object of the invention to disclose the vaginally administrable tablet wherein the tablet is configured for administration in a daily unit dosage of between about 5 mg to about 30 mg mifepristone.

It is another object of the invention to disclose the vaginally administrable tablet wherein the tablet is configured for administration in a daily unit dosage of less than about 5 mg mifepristone.

It is another object of the invention to disclose the vaginally administrable tablet wherein the tablet is additionally provided with a conventional applicator suitable for vaginal administration.

It is a further object of the invention to provide a kit useful for treating leiomyomata leiomyoma, myoma, uterine fibroids, endometriosis, adenomyosis and other uterine disorders. The kit comprising: a plurality of tablets; an applicator for vaginal administration; and, instructions for use of the tablets and the applicator. The aforementioned tablet comprises mifepristone, at least one non-effervescent excipient or diluent, and at least one effervescent excipient,
wherein said tablet is formulated in an immediate release form.

It is another object of the invention to disclose the detailed above kit wherein the tablet has a disintegration time of less than about 15 minutes in the vagina.

It is another object of the invention to disclose the detailed above kit wherein the tablet has disintegration time of between about 15 minutes and about 60 minutes in the vagina.

It is another object of the invention to disclose the detailed above kit wherein the tablet has a disintegration time of less than about 3 minutes in water at room temperature.

It is another object of the invention to disclose the detailed above kit wherein the tablet has disintegration time of between about 3 minutes and about 15 minutes in water at room temperature.

It is another object of the invention to disclose the detailed above kit wherein the tablet comprises between about 0.1 to about 50 mg of mifepristone and between about 5 to about 12 wt.% effervescent excipient.

It is another object of the invention to disclose the detailed above kit wherein the tablet comprises between about 5 to about 20 mg of mifepristone.

It is another object of the invention to disclose the detailed above kit wherein the tablet comprises between about 6 to 8 wt. % effervescent excipient.

It is another object of the invention to disclose the detailed above kit wherein the tablet is configured for administration in a daily unit dosage of between about 5 mg to about 30 mg mifepristone.

It is another object of the invention to disclose the detailed above kit wherein the tablet is configured for administration in a daily unit dosage of less than about 5 mg mifepristone.

It is a further object of the invention to provide a tablet for vaginal administration comprising mifepristone, prepared by the steps of: preparing a mixture consisting of mifepristone and at least one pharmaceutically acceplable non-effervescent excipient or diluent and at least one effervescent excipient wherein the tablet is in an immediate release form and forming a tablet by direct compaction of the mixture the tablet.

It is another object of the invention to disclose the tablet for vaginal administration prepared by the detailed above steps wherein the mixture is further prepared by the steps of: preparing a first mixture consisting of water and the mifepristone, to obtain wetted mifepristone; and, drying the wetted mifepristone to obtain dry mifepristone and mixing the dry mifepristone with at least one pharmaceutically acceptable excipient or diluent to form a second mixture.

It is another object of the invention to disclose the tablet for vaginal administration prepared by the detailed above steps wherein the first mixture additionally comprises a pharmaceutically acceptable excipient.

It is another object of the invention to disclose the tablet for vaginal administration prepared by the detailed above steps wherein the second mixture additionally comprises an effervescent excipient.

It is another object of the invention to disclose the tablet for vaginal administration prepared by the detailed above steps wherein the tablet is useful for treating uterine leiomyomata leiomyoma, myoma, uterine fibroids, endometriosis, adenomyosis and other related disorders, the tablet comprising mifepristone, at least one non-effervescent excipient or diluent, and at least one effervescent excipient.

It is another object of the invention to disclose the tablet for vaginal administration prepared by the detailed above steps wherein the tablet has a disintegration time of less than 15 minutes in the vagina.

It is another object of the invention to disclose the tablet for vaginal administration prepared by the detailed above steps wherein the tablet has disintegration time of between 15 minutes and about 60 minutes in the vagina.

It is another object of the invention to disclose the tablet for vaginal administration prepared by the detailed above steps wherein the tablet has a disintegration time of less than 3 minutes in water at room temperature.

It is another object of the invention to disclose the tablet for vaginal administration prepared by the detailed above steps wherein the tablet has disintegration time of between 3 minutes and about 15 minutes in water at room temperature.

It is another object of the invention to disclose the tablet for vaginal administration prepared by the detailed above steps wherein the tablet comprising between about 0.1 to about 50 mg of mifepristone and between about 5 to about 12 wt.% effervescent excipient.

It is another object of the invention to disclose the tablet for vaginal administration prepared by the detailed above steps wherein the tablet comprising between about 5 to about 20 mg of mifepristone.

It is another object of the invention to disclose the tablet for vaginal administration prepared by the detailed above steps wherein the tablet comprising between about 6 to 8 wt. % effervescent excipient.

It is another object of the invention to disclose the tablet for vaginal administration prepared by the detailed above steps wherein the tablet is administered in a daily unit dosage of between about 5 mg to about 30 mg mifepristone.

It is another object of the invention to disclose the tablet for vaginal administration prepared by the detailed above steps wherein the tablet is administered in a daily unit dosage of less than about 5 mg mifepristone.

It is another object of the invention to disclose the tablet for vaginal administration prepared by the detailed above steps wherein the tablet is additionally provided with a conventional applicator suitable for vaginal administration.

Further described is a method for treating uterine disorders especially uterine leiomyomata leiomyoma, myoma, uterine fibroids, endometriosis, adenomyosis and other related disorders, comprising steps of: obtaining a vaginally administrable tablet, the tablet comprising mifepristone, at least one non-effervescent excipient or diluent, and at least one effervescent excipient; and, administering the tablet vaginally at a therapeutically effective dosage.

Further described is the method for treating uterine disorders wherein the tablet is prepared by steps of: preparing a mixture consisting of mifepristone and at least one pharmaceutically acceptable non-effervescent excipient or diluent and at least one effervescent exepient; and forming a tablet by direct compaction of the mixture.

Further described is the method for treating uterine disorders wherein the mixture is further prepared by steps of: preparing a first mixture consisting of water and the mifepristone, to obtain wetted mifepristone; and, drying the wetted mifepristone to obtain dry mifepristone; and, mixing the dry mifepristone with at least one pharmaceutically acceptable excipient or diluent to form a second mixture.

Further described is the method for treating uterine disorders wherein the second mixture further comprises at least one effervescent excipient.

Further described is the method for treating uterine disorders wherein the tablet is in an immediate release form.

Further described is the method for treating uterine disorders wherein the tablet has a disintegration time of less than 15 minutes in the vagina.

Further described is the method for treating uterine disorders wherein the tablet has disintegration time of between about 15 and about 60 minutes in the vagina.

Further described is the method for treating uterine disorders wherein the tablet has a disintegration time of less than 3 minutes in water at room temperature.

Further described is the method for treating uterine disorders wherein the tablet has disintegration time of between about 3 minutes and about 15 minutes in water at room temperature.

Further described is the method for treating uterine disorders wherein the tablet comprises between about 0.1 to about 50 mg of mifepristone and between about 5 to about 12 wt.% effervescent excipient.

Further described is the method for treating uterine disorders wherein the tablet comprises between about 5 mg to about 20 mg of mifepristone.

Further described is the method for treating uterine disorders wherein the tablet comprises between about 6 to 8 wt. % effervescent excipient.

Further described is the method for treating uterine disorders wherein the method further comprises steps of administering the tablet in a daily unit dosage of between about 5 mg to about 30 mg of mifepristone.

Further described is the method for treating uterine disorders wherein the method further comprises steps of administering the tablet in a daily unit dosage of less than about 5 mg of mifepristone.

Further described is the method for treating uterine disorders wherein the method further comprises steps of applying the tablet by a conventional applicator suitable for vaginal administration.

### BREIF DESCRIPTION OF THE DRAWINGS

In order to understand the invention and to see how it may be implemented in practice, a plurality of embodiments is adapted to now be described, by way of nonlimiting example only, with reference to the accompanying drawings, wherein:

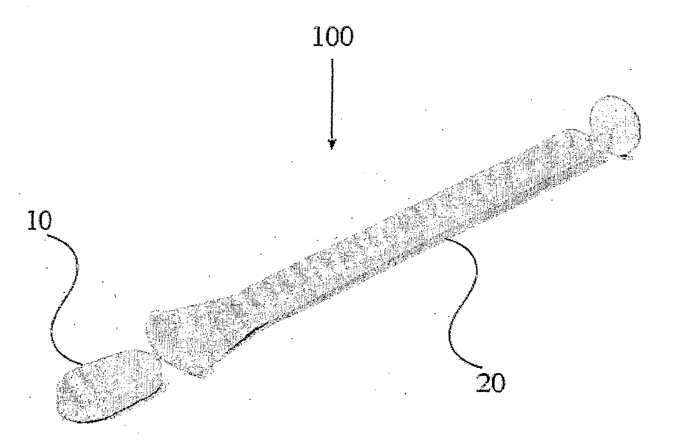

**Fig.1** is an illustration of preferred embodiments of the invention.

### DETAILED DESCRIPTION OF THE INVENTION

The following description is provided, alongside all chapters of the present invention, so as to enable any person skilled in the art to make use of said invention and sets forth the best modes contemplated by the inventor of carrying out this invention. Various modifications, however, is adapted to remain apparent to those skilled in the art, since the generic principles of the present invention have been defined specifically to provide means of treating Uterine disorders, especially leiomyomata, leiomyoma, myoma, uterine fibroids, endometriosis, adenomyosis by introducing a therapeutic composition vaginally.

A specific composition is provided for a tablet designed to be administered intravaginally to uterine leiomyomata and at the endometrium. It is a core principle of the invention to provide the tablet composition especially suitable for the vaginal and uterine area since the tablet is formulated to dissolve immediately in the vaginal environment. The composition contains an effervescent component providing a short disintegration time, effective release and a rapid uptake of the active ingredients in the vaginal and uterine area. The composition is further adapted to the vaginal and uterine environment in that no tablet residues remain after each treatment period. The delivery of the drug, intra vaginally as herein disclosed, is directed close to the disease site and is thus less systemic than orally administered drugs or compositions. Since the method of delivery is relatively localized, lower doses may be given to the patient, whilst maintaining the therapeutic effect.

Mifepristone is a synthetic steroid with antiprogestational effects. Treatment with orally administrable mifepristone, is associated with reduction in uterine and uterine fibroids size and improvement in uterine fibroids symptoms with mifepristone doses of 5mg - 50mg. To date, mifepristone is approved in several countries for use in four indications: early termination of pregnancy (TOP), cervical dilatation prior, to surgical TOP, preparation for prostaglandin- induced TOP during the second trimester and expulsion of a dead fetus during the third trimester.

Vaginal mifepristone is a noninvasive therapy that represents a viable alternative to other medical therapies with fewer side effects.

Vaginal administration has several benefits. The vaginal area is characterized by a rich blood supply, resulting in rapid and steady uptake of drugs, lower and steadier serum concentrations than oral drug delivery and the absence of liver metabolism, which allows low doses with fewer side effects.

Long-term, low-dose mifepristone can be given to perimenopausal women with large, symptomatic uterine fibroids until menopause, when the uterine fibroids typically regress.

In accordance with the invention, a vaginally administrable tablet comprising mifepristone, at least one non-effervescent excipient or diluent, and at least one effervescent excipient, is provided. The aforementioned tablet is formulated in an immediate release form.

The term **'immediate release'** used hereinafter refers to the effervescent properties of the vaginally administrable tablet, having a disintegration time of less than about 3 minutes in water at room temperature or less than about 15 minutes in the vagina.

In accordance with another embodiment of the present invention, the vaginally administrable tablet has an intermediate disintegration time of between about 3 minutes and about 15 minutes in water at room temperature; and, between about 15 minutes and about 60 minutes in the vagina.

In accordance with another embodiment of the present invention the tablet for vaginal administration comprising between about 0.1 to about 50 mg of mifepristone and between about 5 to about 12 wt.% effervescent excipient.

In accordance with another embodiment of the present invention the tablet for vaginal administration comprises between about 5 to about 20 mg of mifepristone.

In accordance with another embodiment of the present invention the tablet for vaginal administration comprises between about 6 to about 8 wt. % effervescent excipient. In accordance with another embodiment of the present invention the tablet for vaginal administration is administered in a daily unit dosage of between about 5 mg to about 30 mg mifepristone.

In accordance with another embodiment of the present invention the tablet for vaginal administration is administered in a daily unit dosage of less than about 5 mg mifepristone.

In accordance with another embodiment of the present invention the tablet for vaginal administration is additionally provided with a conventional applicator suitable for vaginal administration.

Reference is made to a kit useful for treating leiomyomata leiomyoma, myoma, uterine fibroids, endometriosis, adenomyosis and other related disorders. The kit comprises (a) a plurality of tablets (b) an applicator for vaginal administration; and (c) instructions for use of the tablets and the applicator. Each tablet in the aforementioned kit comprises mifepristone, at least one non-effervescent excipient or diluent, and at least one effervescent excipient, wherein

the tablet of the above defined kit is in an immediate release form.

In accordance with another embodiment of the present invention the tablet of the above defined kit has a disintegration time of less than about 15 minutes in the vagina.

In accordance with another embodiment of the present invention the tablet of the above defined kit has a disintegration time of between about 15 minutes and about 60 minutes in the vagina.

In accordance with another embodiment of the present invention the tablet of the above defined kit has a disintegration time of less than about 3 minutes in water at room temperature.

In accordance with another embodiment of the present invention the tablet of the above defined kit has a disintegration time of between about 3 minutes and about 15 minutes in water at room temperature.

In accordance with another embodiment of the present invention the tablet of the above defined kit comprises between about 0.1 to about 50 mg of mifepristone and between about 5 to about 12 wt. % effervescent excipient.

In accordance with another embodiment of the present invention the tablet of the above defined kit comprises between about 5 to about 20 mg of mifepristone.

In accordance with another embodiment of the present invention the tablet of the above defined kit comprises between about 6 to about 8 wt. % effervescent excipient.

In accordance with another embodiment of the present invention the tablet of the above defined kit is configured for administration in a daily unit dosage of between about 5 mg to about 30 mg mifepristone.

In accordance with another embodiment of the present invention the tablet of the above defined kit is configured for administration in a daily unit dosage of less than about 5 mg mifepristone.

Reference is made to a tablet for vaginal administration comprising mifepristone wherein the tablet is formulated in an immediate release form. This tablet is prepared by a method which includes preparing a mixture consisting of water and mifepristone with or without an additional pharmaceutically acceptable excipient to obtain wetted mifepristone. The wetted mifepristone is dried to obtain mifepristone. This first mixture is mixed with at least one pharmaceutically acceptable excipient or diluent to form a second mixture. A tablet is then formed by direct compaction of the second mixture.

A further embodiment of the vaginally administered tablet comprising mifepristone is referenced herein. The aforementioned tablet is prepared by (a) preparing a mixture consisting of mifepristone and at least one pharmaceutically acceptable non-effervescent excipient or diluent with at least one effervescent exepient. (b) the aforementioned tablet is formed by direct compaction of the mixture.

A further embodiment of the vaginally administered tablet comprising mifepristone is referenced herein. The aforementioned mixture is made by mixing water with mifepristone to obtain wetted mifepristone in the absence of a pharmaceutically acceptable excipient or diluent. The aforementioned wetted mifepristone is dried to form dry mifepristone. The aforementioned dry mifepristone is mixed with at least one pharmaceutically acceptable non effervescent excipient or diluent and at least one effervescent excipient to form a mixture. The mixture is formed into a tablet by direct compaction of the mixture.

A further embodiment of the vaginally administered tablet prepared by the above defined steps is referenced herein. The tablet is useful for treating uterine leiomyomata leiomyoma, myoma, uterine fibroids, endometriosis, adenomyosis and other related disorders. The aforementioned tablet comprises mifepristone, at least one non-effervescent excipient or diluent, and at least one effervescent excipient.

A further embodiment of the vaginally administered tablet prepared by the above defined steps is having a disintegration time of less than 15 minutes in the vagina.

A further embodiment of the vaginally administered tablet prepared by the above defined steps is having disintegration time of between about 15 minutes and about 60 minutes in the vagina.

A further embodiment of the vaginally administered tablet prepared by the above defined steps is having disintegration time of less than 3 minutes in water at room temperature.

A further embodiment of the vaginally administered tablet prepared by the above defined steps is having disintegration time of between about 3 minutes and about 15 minutes in water at room temperature.

A further embodiment of a tablet for vaginal administration comprising mifepristone is referenced herein. The tablet, prepared by the above defined steps comprises between about 0.1 to about 50 mg of mifepristone and between about 5 to about 12 wt.% effervescent excipient.

A further embodiment of a tablet for vaginal administration comprising mifepristone is referenced herein. The tablet, prepared by the above defined steps comprises between about 5 to about 20 mg of mifepristone.

A further embodiment of a tablet for vaginal administration comprising mifepristone is referenced herein. The tablet, prepared by the above defined steps comprises between about 6 to 8 wt. % effervescent excipient.

A further embodiment of a tablet for vaginal administration comprising mifepristone is referenced herein. The tablet, prepared by the above defined steps is administered in a daily unit dosage of between about 5 mg to about 30 mg mifepristone.

A further embodiment of a tablet for vaginal administration comprising mifepristone is referenced herein. The tablet, prepared by the above defined steps is administered in a daily unit dosage of less than about 5 mg mifepristone.

A further embodiment of a tablet for vaginal administration comprising mifepristone is referenced herein. The tablet, prepared by the above defined steps additionally provided with a conventional applicator suitable for vaginal administration.

Reference is now made to a method for treating uterine disorders especially Uterine Leiomyomata, leiomyoma, myoma, uterine fibroids, endometriosis, adenomyosis and other related disorders. The method comprises obtaining a vaginally administrable tablet comprising mifepristone, ay least one non-effervescent excipient or diluent, and at least one effervescent excipient; and administering the tablet vaginally at a therapeutically effective dosage.

Reference is now made to the method for treating uterine disorders as defined above. The method comprises obtaining a tablet for vaginal administration comprising mifepristone. The tablet is prepared by the steps of preparing a mixture consisting of mifepristone and at least one pharmaceutically acceptable non-effervescent excipient or diluent and at least one effervescent exepient; and, forming a tablet by direct compaction of the mixture.

Reference is now made to the method for treating uterine disorders as defined above, wherein the mixture is further prepared by steps of preparing a first mixture consisting of water and said mifepristone, to obtain wetted mifepristone; and, drying said wetted mifepristone to obtain dry mifepristone; and, mixing said dry mifepristone with at least one pharmaceutically acceptable excipient or diluent to form a second mixture.

Reference is now made to the method for treating uterine disorders as defined above, wherein the second mixture further comprises at least one effervescent excipient.

Reference is now made to the method for treating uterine disorders as defined above, wherein the tablet is in an immediate release form.

Reference is now made to the method for treating uterine disorders as defined above, wherein the tablet has a disintegration time of less than about 15 minutes in the vagina.

Reference is now made to the method for treating uterine disorders as defined above, wherein the tablet has disintegration time of between about 15 minutes and about 60 minutes in the vagina.

Reference is now made to the method for treating uterine disorders as defined above, wherein the tablet has a disintegration time of less than about 3 minutes in water at room temperature.

Reference is now made to the method for treating uterine disorders as defined above, wherein the tablet has disintegration time of between about 3 minutes and about 15 minutes in water at room temperature.

Reference is now made to the method for treating uterine disorders as defined above, wherein the tablet comprises between about 0.1 to about 50 mg of mifepristone and between about 5 to about 12 wt.% effervescent excipient.

Reference is now made to the method for treating uterine disorders as defined above, wherein the tablet comprises between about 5 mg to about 20 mg of mifepristone.

Reference is now made to the method for treating uterine disorders as defined above, wherein the tablet comprises between about 6 to 8 wt. % effervescent excipient.

Reference is now made to the method for treating uterine disorders as defined above, wherein the method further comprises steps of administering said tablet in a daily unit dosage of between about 5 mg to about 30 mg of mifepristone.

Reference is now made to the method for treating uterine disorders as defined above, wherein the method further comprises steps of administering said tablet in a daily unit dosage of less than about 5 mg of mifepristone.

Lastly, reference is now made to the method for treating uterine disorders as defined above, wherein the method further comprises steps of applying said tablet by a conventional applicator suitable for vaginal administration.

In order to understand the invention and to see how it may be implemented in practice, a plurality of preferred embodiments will now be described, by way of nonlimiting example only, with reference to the following examples.

### EXAMPLE 1

### Preparation of vaginal tablets containing mifepristone

Mifepristone vaginal tablets contain: 10 mg mifepristone and the following inactive ingredients: Lactose, starch, citric acid, sodium bicarbonate as an effervescent excipient, PVP and magnesium stearate.

Each mifepristone vaginal tablet comprising the following ingredients:

| | | |
|---|---|---|
| 1. Mifepristone | 10 | mg |
| 2. Lactose | 693 | mg |
| 3. Starch 1500 | 168 | mg |
| 4. Citric acid | 45.6 | mg |
| 5. Sodium bicarbonate | 34.4 | mg |
| 6. PVP k30 | 3.9 | mg |
| 7. Magnesium stearate | 10 | mg |

The preparation of 4000 vaginal tablets includes the following steps:

**Table 1: preparation of 4000 vaginal tablets**

| **Step.** | **Mix Ingredients** | **Mixing Time (min)** | **Mesh** |
|---|---|---|---|
| A | 3 + 6 + 1 | 2' | 250 mesh |
| B | A + 2 (½) | 2' | |
| C | 4 | | 250 mesh |
| D | B+C | 2' | |
| E | 5 | | 250 mesh |
| F | D+E | 2' | |
| G | F + 2 (½) | 2' | |
| H | 7 | | 250 mesh |
| I | G+H | 8' | |
| G | compaction of the mixture into tablets | | |
| H | counting tablets | | |

### EXAMPLE 2

**The effect of vaginal mifepristone on the reduction of uterine fibroids size and the symptoms associated with the fibroids**

The vaginal tablets of the present invention were subjected to phase II clinical trials:
The drug is manufactured by Floris according to GMP (Good Manufacturing Practice).

A protocol for clinical trial is herein described:

| | |
|---|---|
| **Sponsor:** | **Bio-pro medical Ltd.** |
| | **Ilan Lapidot** |
| | **Hashita 8 St. Industrial park Caesarea 38900** |
| | **il@lapidot.com** |
| | **Tel: 04-6309603 Fax:04-6309642** |
| | |
| **Monitor:** | **Shlomit Cohen** |
| | **Hashita 8 St. Industrial park Caesarea 38900** |
| | **Shlomitc@lapidot.com** |
| | **Tel: 04-6309630 Fax:04-6300642.** |
| | |
| **Principal Investigator:** | **Prof. Seidman** |
| | **Shiba medical center** |
| | **Tel-Hashomer** |
| | **Tel: 03-5302697 Fax:03-5352081** |
| | |
| **Medical Monitor:** | **Dr. Daniel katz** |
| | **Hashita 8 St. Industrial park Caesarea 38900** |
| | **Daniel.Katz@Lapidot.com** |

### Rationale of the clinical trial:

Treatment with mifepristone, an antiprogestin, is associated with reduction in uterine and uterine fibroids size and improvement in uterine fibroids symptoms following administration of oral mifepristone doses of 5mg - 50mg.

Murphy¹¹ et all demonstrated a clinically significant regression in the size of uterine leiomyomata using 5, 25, and 50 mg of mifepristone daily for 12 weeks of therapy.

Yang⁸ et all studied 10 mg and 20 mg of mifepristone administered daily for 12 weeks in women with large leiomyomata. Reduction in volume was equivalent in the two groups and was comparable to the shrinkage found by Murphy.

Vaginal mifepristone is noninvasive therapy that represents a viable alternative to other medical therapies. Vaginally low doses are expected to yield reduction in uterine fibroids size and improvement in uterine fibroids symptoms with fewer side effects than in oral doses.

Vaginal administration is characterized in rapid uptake, lower and steadier serum concentrations than oral drug delivery and the absence of liver metabolism, which allows low doses with fewer side effects.

### Objectives of the clinical trial

The primary objective of the study is to evaluate the effect of daily dosage of vaginally mifepristone on reduction of uterine fibroids size.

Secondary objectives are to evaluate the effect of daily dosage of vaginally mifepristone on uterine size, the symptoms associated with uterine fibroids and to assess whether the drug improves side effects and quality of life.

### Parameters for evaluation of the results (endpoint)

### Endpoints:

### Primary end point:

### Efficacy:

- Decrease of uterine fibroids volume by 25% and up - will be assessed by transabdominal and transvaginal Ultrasonography (depending on uterine fibroids size) at baseline, monthly during the course of therapy and at the final follow up.

### Secondary end points:

### Efficacy:

- Decrease of uterus volume - will be assessed by transabdominal and transvaginal Ultrasonography (depending on uterine fibroids size) at baseline, monthly during the course of therapy and at the final follow up.
- Symptoms of uterine fibroids - assessed using "Uterine Fibroid Symptoms Quality of Life Questionnaire"⁴ at baseline, monthly during the course of therapy and at the final follow up.

### Safety:

- Safety will be assessed by Pipelle test (endometrial sampling) prior to starting the study and at 3-months.
- Safety will be additionally assessed by laboratory tests - serum concentrations of hemoglobin levels, liver function (liver enzymes SGOT & SGPT) and renal function (creatinine & urea).

### Patient Selection

### Subjects - Number of subjects, age and medical condition.

30 healthy women, age 30-53, diagnosed with symptomatic uterine fibroids.

### Inclusion Criteria

- Age 30-53 years old.
- Uninterested in further fertility.
- Premenopausal status.
- Good general health.
- Active symptoms related to uterine fibroids.
- Subjects that were interested in noninvasive treatment.
- Hemoglobin value greater than 9 g/dL.
- Subjects that had not used hormonal medications in the past 6 months.
- Subjects that agreed to complete a monthly symptom questionnaire and to undergo ultrasonography examinations each month and at study completion:
- Subjects that agreed to undergo pipelle test at study completion.
- Subjects that provided informed consent and agree to comply with all study procedures.
- Subjects will obligate to use non hormonal contraceptives.
- Negative PAP results from last year.

### Exclusion Criteria

- Pregnancy or active attempts to become pregnant.
- Severe anemia.
- Menopausal status.,
- Abnormal liver function (liver function tests greater than 1.5 times upper range of normal).
- Abnormal renal function (serum creatinine > 1.5 mg/dl).
- Participants with significant increase in uterine fibroids size during a short time.
- Exclusionary health problems contraindicating mifepristone included adrenal disease; sickle cell anemia; severe liver, respiratory, or renal disease; and blood clotting defect.
- Current use of steroids, "anticoagulants, herbals, or botanicals with possible hormonal effects.
- Oral contraception or hormone replacement therapy within the last 3 months.
- GnRH analogues or depomedroxyprogesterone within the last 6 months.

### Withdrawal of subjects - Conditions for discontinuation of the clinical trial

Subjects will be withdrawn from the study:
- If they are experiencing a serious adverse event felt to be related to study drug.
- If it is deemed by the principal investigator to be in their best interest.
- If they exhibit low compliance in some/all stages (drug administration, questionnaire, examinations), lost to follow up, patient's decision.

All participants in the study will be included in the final study analyses.

Reasons why subjects are discontinued from the clinical trial will be documented on the Study Termination Form, along with any referrals that are made.

### Study Procedure Descriptions

### Study design

### Screening Procedures:

- Subjects will be recruited from symptomatic uterine fibroids women (total of 30).
- Signing the informed consent form.
- Subjects must meet all inclusion and none of the exclusion criteria.
- Subjects will be inquired regarding their medical history and their demography.
- Concomitant medication - all concomitant medications taken during study participation will be recorded in source document.
- Subjects will be inquired regarding their last period date.
- Physical Examination of vaginal, heart and lungs.
- Vital signs - blood pressure, pulse, fever.
- Laboratory tests - hemoglobin levels, liver function (liver enzymes SGOT & SGPT) and renal function (creatinine & urea).
- Uterine fibroids and uterus - will be assessed by transabdominal or by transvaginal Ultrasonography (depending on uterine fibroids size).The uterus will be measured in three planes and a total volume calculated. The five largest uterine fibroids will be identified, a volume calculated for each of the uterine fibroids, and the results summed. Baseline uterine volume will be subtracted from each subsequently measured uterine volume, and volume changes will be analyzed.

### Baseline (up to 21 days from screening):

- Subjects must meet all inclusion and none of the exclusion criteria.
- Concomitant medication - all concomitant medications taken during study participation will be recorded in source document.
- Subjects will be inquired regarding their last period date.
- Physical Examination of vaginal, heart and lungs.
- Vital signs - blood pressure, pulse, fever.
- Pregnancy test will be preformed - females of reproductive potential must have a negative serum pregnancy test on whole course of administration of the study agent.
- Pipelle test (endometrial sampling) will be preformed.
- Filling out the symptom questionnaire - the questionnaire will be filled out during the visit in site. Symptoms of uterine fibroids will be assessed by using "Uterine Fibroid Symptoms Quality of Life Questionnaire"⁴.
   The symptom Quality of Life questionnaire includes:
   Primary scale - measures overall quality of life, scale of 1-100, with higher scores indicating better quality of life.
   Secondary scales - measure perceived impact of uterine fibroids on activities of daily living, general concern and worry, energy and mood, sense of self-control, self-consciousness, and sexual functioning.
- Administration of Mifepristone- subjects will be receiving 33 vaginal tablets which will be administrated once daily. The tablets will be administrated by using a multi-dose applicator.

### Follow up 1 -30 ± 3 days of treatment

- Concomitant medication - all concomitant medications taken during study participation will be recorded in source document.
- Subjects will be inquired regarding their last period date.
- Physical Examination of vaginal, heart and lungs.
- Vital signs - blood pressure, pulse, fever.
- Laboratory tests - hemoglobin levels, liver function (liver enzymes SGOT & SGPT) and renal function (creatinine & urea).
- Pregnancy test will be preformed - females of reproductive potential must have a negative serum pregnancy test on whole course of administration of the study agent.
- Uterine fibroids and uterus - will be assessed by transabdominal or by transvaginal Ultrasonography (depending on uterine fibroids size). The uterus will be measured in three planes and a total volume calculated. The five largest uterine fibroids will be identified, a volume calculated for each of the uterine fibroids, and the results summed. Baseline uterine volume will be subtracted from current subsequently measured uterine volume, and volume changes will be analyzed.
- Filling out the symptom questionnaire - the questionnaire will be filled out during the visit in site. Symptoms of uterine fibroids will be assessed by using "Uterine Fibroid Symptoms Quality of Life Questionnaire"⁴.
   The symptom Quality of Life questionnaire includes:
   Primary scale - measures overall quality of life, scale of 1-100, with higher scores indicating better quality of life.
   Secondary scales - measure perceived impact of uterine fibroids on activities of daily living, general concern and worry, energy and mood, sense of self-control, self-consciousness, and sexual functioning.
- Administration of Mifepristone- subjects will be receiving 33 vaginal tablets which will be administrated once daily. The tablets will be administrated by using a multi-dose applicator.
- Return of mifepristone remaining tablets.
- Subjects will be inquired regarding adverse effects during treatment.

### Follow up 2 -60 ± 3 days of treatment

- Concomitant medication - all concomitant medications taken during study participation will be recorded in source document.
- Subjects will be inquired regarding their last period date.
- Physical Examination of vaginal, heart and lungs.
- Vital signs - blood pressure, pulse, fever.
- Laboratory tests - hemoglobin levels, liver function (liver enzymes SGOT & SGPT) and renal function (creatinine & urea).
- Pregnancy test will be preformed - females of reproductive potential must have a negative serum pregnancy test on whole course of administration of the study agent.
- Uterine fibroids and uterus - will be assessed by transabdominal or by transvaginal Ultrasonography (depending on uterine fibroids size).The uterus will be measured in three planes and a total volume calculated. The five largest uterine fibroids will be identified, a volume calculated for each of the uterine fibroids, and the results summed. Baseline uterine volume will be subtracted from current subsequently measured uterine volume, and volume changes will be analyzed.
- Filling out the symptom questionnaire - the questionnaire will be filled out during the visit in site. Symptoms of uterine fibroids will be assessed by using "Uterine Fibroid Symptoms Quality of Life Questionnaire"⁴.
   The symptom Quality of Life questionnaire includes:
   Primary scale - measures overall quality of life, scale of 1-100, with higher scores indicating better quality of life.
   Secondary scales - measure perceived impact of uterine fibroids on activities of daily living, general concern and worry, energy and mood, sense of self-control, self-consciousness, and sexual functioning.
- Administration of Mifepristone- subjects will be .receiving 33 vaginal tablets which will be administrated once daily. The tablets will be administrated by using a multi-dose applicator.
- Return of mifepristone remaining tablets.
- Subjects will be inquired regarding adverse effects during treatment.

### Follow up 3 -90 ± 3 days of treatment

- Concomitant medication - all concomitant medications taken during study participation will be recorded in source document.
- Subjects will be inquired regarding their last period date.
- Physical Examination of vaginal, heart and lungs.
- Vital signs - blood pressure, pulse, fever.
- Laboratory tests - hemoglobin levels, liver function (liver enzymes SGOT & SGPT) and renal function (creatinine & urea).
- Pharmacokinetics test- mifepristone serum levels (assay).
- Uterine fibroids and uterus - will be assessed by transabdominal or by transvaginal Ultrasonography (depending on uterine fibroids size).The uterus will be measured in three planes and a total volume calculated. The five largest uterine fibroids will be identified, a volume calculated for each of the uterine fibroids, and the results summed. Baseline uterine volume will be subtracted from current subsequently measured uterine volume, and volume changes will be analyzed.
- Filling out the symptom questionnaire - the questionnaire will be filled out during the visit in site. Symptoms of uterine fibroids will be assessed by using "Uterine Fibroid Symptoms Quality of Life Questionnaire"⁴.
   The symptom Quality of Life questionnaire includes:
   Primary scale - measures overall quality of life, scale of 1-100, with higher scores indicating better quality of life.
   Secondary scales - measure perceived impact of uterine fibroids on activities of daily living, general concern and worry, energy and mood, sense of self-control, self-consciousness, and sexual functioning.
- Pipelle test (endometrial sampling) will be preformed.
- Return of mifepristone remaining tablets.
- Subjects will be inquired regarding adverse effects during treatment.

### Follow up 4/final -180 ± 7 days of treatment

- Physical Examination of vaginal, heart and lungs.
- Subjects will be inquired regarding their last period date.
- Vital signs - blood pressure, pulse, fever.
- Laboratory tests - serum concentrations of hemoglobin levels, liver function (liver enzymes SGOT & SGPT) and renal function (creatinine & urea).
- Uterine fibroids and uterus - will be assessed by transabdominal or by transvaginal Ultrasonography (depending on uterine fibroids size).The uterus will be measured in three planes and a total volume calculated. Baseline uterine volume will be subtracted from current subsequently measured uterine volume, and volume changes will be analyzed. Current measurement will be compared with previous measurement (follow up 3 - end o treatment).
- Filling out the symptom questionnaire - the questionnaire will be filled out during the visit in site. Symptoms of uterine fibroids will be assessed by using "Uterine Fibroid Symptoms Quality of Life Questionnaire"⁴.
   The symptom Quality of Life questionnaire includes:
   Primary scale - measures overall quality of life, scale of 1-100, with higher scores indicating better quality of life.
   Secondary scales - measure perceived impact of uterine fibroids on activities of daily living, general concern and worry, energy and mood, sense of self-control, self-consciousness, and sexual functioning.

### Schedule of procedures by visit

| ***Visit number*** | **Visit 1** | **Visit 2** | **Visit 3** | **Visit 4** | **Visit 5** | **Visit 6** |
|---|---|---|---|---|---|---|
| ***Visit name*** | **Screening** | **Baseline** | **Follow up 1** | **Follow up 2** | **Follow up 3** | **Follow up 4** |
| ***Day in study*** | **Up to 21 days before baseline** | **0** | **30±3** | **60±0** | **90±3** | **180±3** |
| Informed consent | √ | | | | | |
| Inclusion / exclusion criteria | √ | √ | | | | |
| Subject demography | √ | | | | | |
| Medical history | √ | | | | | |
| Concomitant medication | √ | | √ | √ | √ | |
| Date of last period | √ | √ | √ | √ | √ | √ |
| Physical Examination | √ | √ | √ | √ | √ | √ |
| Vital signs | √ | √ | √ | √ | √ | √ |
| Laboratory tests | √ | | √ | √ | √ | √ |
| Pharmacokinetics test | | | | | √ | |
| Pregnancy test | | √ | √ | √ | | |
| Ultrasound | √ | | √ | √ | √ | √ |
| Symptoms questionnaire | | √ | √ | √ | √ | √ |
| Pipelle test | | √ | | | √ | |
| Administration of mifepristone | | √ | √ | √ | | |
| Adverse Events | | | √ | √ | √ | |

### Study Drug and Dosages

### Study Drug Testing, Formulation and Dosages

Mifepristone 10mg will be administrated vaginally once daily for three months. Mifepristone vaginal tablets contain mifepristone 10 mg and the following inactive ingredients: Lactose, starch, adipic acid, sodium bicarbonate, PVP, magnesium stearate, sodium lauryl sulphate, silicium dioxide.

Study drug will be manufactured by Floris according to GMP.
The dose of mifepristone selected for this study (10 mg/day) is based on the dose used in the oral treatment of uterine fibroids (dose range: 5 - 50mg/day).
Mifepristone vaginal preparation contains the same excipients as Endometrin vaginal tablets (listed drug), therefore the excipients safety is already known.
Endometrin, listed drug (1999) is indicated as progesterone supplementation or replacement in case such as treatment of infertile women and IVF and is given on a dally basis.

### Packaging of Study Drug

HDPE (High-Density Polyethylene) bottle, with vaginal multiple use applicator.
Each bottle contains 33 vaginal tablets (30 for daily treatment of one month, 3 vaginal tablets as reserve).
The applicator will be supplied with the trial drug and will be replaced each month with receiving the trial drug bottle.
This applicator is approved by the Israeli MoH as a part of Endometrin product.

### Storing, Dispensing, and Disposing of Study Drug

Upon receipt of the study treatment supplies from Bio=Pro medical, an inventory will be performed and a drug receipt log filled out and signed by the person accepting the shipment. Any damaged or unused study drug in the shipment will be documented in the study files and in the pharmacy files.
The study drug should be stored in dry place at temperatures range of 15-25°c.
The access to study drug dispensed to the Investigator will be controlled by the Investigator and the Investigator's study staff.
Each participant would receive 1 bottle with 33 tablets at the beginning of the trial and at the first and second month till the end of the trial.
Accountability will be preformed by the hospital pharmacy and monitored by the monitor.
At the completion of the study, there will be a final reconciliation of drug shipped, drug consumed, and drug remaining. This reconciliation will be logged on the drug reconciliation form, signed and dated. Any discrepancies noted will be investigated, resolved, and documented prior to destruction of unused study drug. After appropriate accounting, unused study drug will be destroyed on site with the date of this action documented in the study files.
The drug bottles will be labeled with unique drug codes. The code will include the initials of the subject accompanied by the screening number (two digits 01-30), the enrollment number (two digits 01-30) and the kit number (one digit 1-3) for example: IT0101-1.

### Assessment of Efficacy

1. Reduction of uterine fibroids (25% and up) and uterus - will be assessed by transabdominal and transvaginal Ultrasonography (depending on uterine fibroids size) at baseline, monthly during the course of therapy and at the final follow up. The uterus will be measured in three planes and a total volume calculated.
2. Improvement of symptoms associated with uterine fibroids - assessed using "Uterine Fibroid Symptoms Quality of Life Questionnaire"⁴ at baseline, monthly during the course of therapy and at the final follow up.

### Assessment of Safety

1. Uterus safety (endometrial cells) will be assessed by Pipelle test prior to starting the study and at 3 months.
2. Hemoglobin levels, liver function and renal function will be assessed by laboratory tests of hemoglobin levels, liver function (liver enzymes SGOT & SGPT) and renal function (creatinine & urea).
3. Side effects (such as bleeding and pain) will be evaluation by the symptom Quality of Life questionnaire.

### Adverse Event

### Definition of adverse event

An Adverse Event (AE) is any untoward medical occurrence in a subject administered a pharmaceutical product whether it is related to the pharmaceutical product in question or not.

This includes a change in a subject's condition or laboratory results, which has or could have a deleterious effect on the subject's health or well-being.

### Methods for eliciting adverse events:

All subjects should be carefully monitored for occurrence of AEs during the study and for a reasonable time period thereafter.

All directly observed and spontaneously reported AEs should be recorded in the CRF. In addition, each study subject will be asked an open question about possible AEs at baseline of treatment and at each clinic visit up to final visit (follow up 4).

The following evaluations are to be done by the Investigator in connection with the AE:
- type of event
- seriousness
- degree of severity
- duration (start-end)
- action taken
- likelihood of causality with study product
- outcome of the adverse event

### Definition of Serious Adverse Events:

A Serious Adverse Event (SAE) is defined as any untoward medical occurrence that at any dose fulfills at least one of the following criteria:
- is life-threatening
- results in death
- requires hospitalization, initial or prolonged
- results in persistent or significant disability/incapacity
- is a congenital anomaly/birth defect or
- is regarded as medically important without meeting the above mentioned
- other significant medical events.

### Reporting of Serious Adverse Events:

SAEs must be reported by the Investigator within one working day to the sponsor The Initial report should contain as a minimum the following information:
- subject identification
- treatment specification
- adverse event symptoms/ diagnosis
- time specification for the medical event
- name of the original reporter

A serious Adverse Event Report must also be completed, signed and sent to the sponsor within one working day via fax or regular mail after a direct voice contact.

Apart from the information above, the follow-up report should also contain the following information:
- assessment of severity
- assessment of causality

SAEs should be reported to the sponsor and to the medical monitor even after the clinical study has been finished if, in the judgment of the investigator, there might be an association between the event and the previous use of the study produc or as a result of the study procedures. The post-treatment observation time period will be 90 days after end of treatment.

The investigator is responsible for informing the IIRB/IEC according to local regulations.

### Follow-up period after an adverse event:

After an incidence of an AE, the subject has to be followed up until either the AE has ceased or until the subject is under professional medical care and a potential causality between the study drug and the AE has been penetrated.

Where there was a pregnancy during study treatment the subject and offspring must be monitored for AEs during the entire pregnancy. Any AE occurring to the offspring during pregnancy or at birth must be reported.

The post-treatment safety observation period will be 90 days.

### Clinical Data Collection

### Clinical Data Collection

Source documents- all information, original records of clinical findings, observations, or other activities in a clinical trial will be collected.

All data requested on the case report form (CRF) will be recorded and collected. All missing data must be explained - DCF.

### Monitoring

The progress of a clinical trial is conducted, recorded, and reported in accordance with the protocol, Standard Operating Procedures (SOPs), Good Clinical Practice (GCP), and in accordance with the applicable regulatory requirements.

During the course of the study, the monitor will review cumulative study data approximately every month until the end of the trial. The first monitoring will be conducted not later than two weeks after the study initiation (first subject enrollment). Monitoring will evaluate safety, efficacy, study conduct, and scientific validity and integrity of the trial. As part of this responsibility, monitoring members must be satisfied that the timeliness, completeness, and accuracy of the data submitted to them for review are sufficient for evaluation of the safety and welfare of study subjects. The monitor may also convene as needed if stopping criteria are met or other safety issues arise that the Principal Investigator would like the monitor to address.

### Data recording/Case Report Forms

A CRF (Case Report Form) will be completed for each subject enrolled into the clinical study. The investigator-sponsor will review, approve and sign/date each completed CRF; the investigator-sponsor's signature serving as attestation of the investigator-sponsor's responsibility for ensuring that all clinical and laboratory data entered on the CRF are complete, accurate and authentic.

*Source Data* are the clinical findings and observations, laboratory and test data, and other information contained in *Source Documents. Source Documents* are the original records (and certified copies of original records); including, but not limited to, hospital medical records, physician or office charts, physician or nursing notes, subject diaries or evaluation checklists, pharmacy dispensing records, recorded data from automated instruments, etc. When applicable, information recorded on the CRF shall match the *Source Data* recorded on the *Source Documents.*

### Confidentiality privacy of the participants

The study plan will contain instructions for protecting the privacy of the participants and the confidentiality of the information collected.

Confidentiality of personal details and results received from trial participants are information protected by the right of privacy, The investigators shall restrict access to places where medical information test results of the participant are kept.

Subject names or other directly identifiable information will not appear on any reports, publications, or other disclosures of clinical study outcomes.

### Investigator responsibilities

The principal investigator is responsible for the conduction of the clinical trial at a trial site and for the team at a trial site.

### Statistical Methods and Data Analysis

### Data Management

The data management system will use SAS® version 9.1 with FSEDIT procedure (FSP and AF products).

The CRF's will be collected from the site and will be sent to Data Management by the Study Monitor. The CRF's will be logged and the data will be entered into the study database using double data entry with verification upon second entry. Text items/comments will be entered once and checked manually against the CRF's. Queries will be generated by programmed checks or entered manually. Once the queries are reviewed by quality control, they will be sent to the Monitor for resolution at the investigational site.

### General

All measured variables and derived parameters will be listed individually and, if appropriate, tabulated by descriptive statistics. Descriptive statistics and summary tables will be provided giving sample size (n), absolute and relative frequency of categorical variables and sample size, arithmetic mean, standard deviation, coefficient of variation (if appropriate), median, minimum and maximum, percentiles and 95%. CI (Confidence Interval) for means of continuous variables. All statistical tests applied will be two-tailed and 5% or less will be considered statistically significant.

### Statistical Methods

The following statistical tests will be used to analyze the data in this study:
**Paired T-test or Rank-sign-Test** (as appropriate) will be applied for testing the statistical significance of the changes in volume of uterine fibroids and uterus from baseline to all post baseline measurements.
**Rank -Test or Sign-Rank -Test** (as appropriate) will be applied for testing the statistical significance of the differences in symptoms and menstrual bleeding, as assessed by questionnaire, between baseline and all post baseline time points.
**Paired T-test or Rank-sign-Test** (as appropriate) will be applied for testing the statistical significance of the changes in all measured laboratory tests: Hemoglobin, liver and renal functions from baseline to all post baseline measurements.

Coding of AEs will be performed automatically using the Medical Dictionary for Drug Regulatory Activities (MedDRA) dictionary. Similarly, coding of all medications will occur using the WHO (World Health Organization) Drug dictionary. SAEs will be coded using MedDRA.

All tests applied are two-tailed, and p value of 5% or less will be considered statistically significant. The data will be analyzed using the SAS® software version 9.1 (SAS Institute, Cary, North Carolina).

### Demographic and Baseline Data

Demographic and baseline data will be presented in appropriate tables: Descriptive statistics and summary tables giving sample size (n), absolute and relative frequency for categorical variables and sample size, arithmetic mean, standard deviation, coefficient of variation (if appropriate), median, minimum and maximum, percentiles and 95% CI (Confidence Interval) for means of continuous variables.

### Sample Size & Power of the Study

A one group Chi-square test with a 0.050 two-sided significance level will have 80% power to detect the difference between the Null hypothesis proportion (PI), and the Alternative proportion (PII) (Effect=25%, PI-PII), when the sample size is 30.

### Conclusions

Treatment with mifepristone, an antiprogestin, is associated with reduction in uterine and uterine fibroids size and improvement in uterine fibroids symptoms following oral mifepristone doses of 5mg - 50mg. Vaginal mifepristone administration is noninvasive therapy that represents a viable alternative to other medical therapies. Vaginally low doses are expected to yield reduction in uterine fibroids size and improvement in uterine fibroids symptoms with fewer side effects than in oral doses. Vaginal administration is characterized in rapid uptake, lower and steadier serum concentrations than oral drug delivery and the absence of liver metabolism, which allows low doses with fewer side effects.

### EXAMPLE 3

Reference is now made to Appendix A which is the **certificate of analysis** of the mifepristone vaginal tablets which is incorporated in its entirety as an example.

### REFERENCES

1. Fiscella K, Eisinger SH., Meldrum S, Feng C, Fisher SG., Guzick DS. Effect of Mifepristone for Symptomatic Uterine fibroidsta on Quality of Life and Uterine Size. Obstetrics & Gynecology 2006;108:1381-1387
2. Steinauer J, Pritts EA, Jackson R, Jacoby AF. Systematic review of mifepristone for the treatment of uterine uterine fibroidsta. Obstet Gynecol 2004; 103:1331-6.
3. Eisinger SH, Meldrum S, Fiscella K, le Roux HD, Guzick DS. Low-dose mifepristone for uterine uterine fibroidsta. Obstet Gynecol 2003;101:243-50
4. Spies JB, Coyne K, Guaou GN, Boyle D, Skyrnarz-Murphy K, Gonzalves SM. The UFS-QOL, a new disease-specific symptom and health-related quality of life questionnaire for uterine fibroidsta. Obstet Gynecol 2002;99:290-300
5. Management of uterine fibroids. AHRQ Publication No. 01-E051. Rockville (MD): Agency for Healthcare Research and Quality. Evidence Report/Technology Assessment: Number 34. Available at: http://www.ahrq.gov/clinic/epcsums/utersumm.htm. Retrieved September 14, 2006.
6. Eisinger SH, Bonfiglio T, Fiscella K, Meldrum S, Guzick DS. Twelve-month safety and efficacy of low-dose mifepristone for uterine uterine fibroidss. J Min Invas Gynecol 12:227-33.
7. Murphy AA, Kettel LM, Morales AJ, Roberts VJ, Yen SS. Regression of uterine uterine fibroidsta in response to the antiprogesterone RU 486. J Clin Endocrinol Metab 1993; 76:513-7.
8. Yang Y, Zheng S, Li K. Treatment of uterine uterine fibroids by two different doses of mifepristone. Chin J Obstet Gynecol 1996; 31:624-6.
9. Vollenhoven B. Introduction: The epidemiology of uterine uterine fibroidss. Baillieres Clin Obstet Gynaecol 1998; 12: 169-76.
10. Eisinger SH, Bonfiglio T, Fiscella K, Meldrum S, Guzick DS. Twelve-month safety and efficacy of low-dose mifepristone for uterine uterine fibroidss. J Min Invas Gynecol 2005 12:227-33.
11. Murphy AA, Morales AJ, Kettel LM, Yen SS. Regression of uterine uterine fibroidsta to the antiprogesterone RU486: dose-response effect. Fertil Steril. 1995 Jul; 64(1):187-90.
12. UK SPC 2006 http://www.emc.medicines.ong.uk/emc/assets/c/html/displaydoc.asp?document id=617
13. http://www.drugbank.ca/cgi-bin/getCard.cgi?CARD=DB00834
14. Zeng C, Gu M, Huang H. A clinical control study on the treatment of uterine leiomyoma with gonadotrophin releasing hormone agonist or mifepristone in Chinese_.Zhonghua Fu Chan Ke Za Zhi 1998; 33:490 -2.
15. Mifepristone and misoprostol sequential regimen side effects, complications and safety.
16. Pharmacological properties of mifepristone: toxicology and safety in animal and human studies.
17. Vander Schoot P, Baumgarten R. Effects of treatment of male and female rats in infancy with mifepristone on reproductive function in adulthood. J Reprod Fertil 1990; 90:255-66.
18. Jost A. New data on the hormonal requirements of the pregnant rabbit: partial pregnancies and fetal abnormalities after treatment with a hormonal antagonist at subabortifacient doses. CR Acad Sci 1986; 303(series III, no. 7):281-4.
19. Deraedt R, Vannier B, Fournex R. Toxicological study on RU 486. In: Baulieu EE, Segal SJ, editors. The antiprogestin steroid RU 486 and human fertility control. New York: Plenum Press, 1985. p. 123-6.
20. Brogden RN, Goa KL, Faulds D. Mifepristone. A review of its pharmacodynamic and pharmacokinetic properties, and therapeutic potential. Drugs 1993; 45: 384-409.
21. Kekkonen R, Heikinheimo O, Mandelin E, La "hteenma"kki P. Pharmacokinetics of mifepristone after low oral doses. Contraception 1996; 54:229-34.
22. Moguilewsky M, Philibert D. Biochemical profile of mifepristone. In: Baulieu EE, Segal SJ, editors. The antiprogestin steroid RU 486 and human fertility control. New York7 Plenum Press; 1985. p. 87-97
23. Bertagna X, Bertagna C, Luton JP, Husson JM, Girard F. The new steroid analog RU 486 inhibits glucocorticoid action in man. J Clin Endocrinol Metab 1984; 59:25 - 8
24. Bertagna X, Escourolle H, Pinquier JL, et al. Administration of RU 486 for 8 days in normal volunteers: antiglucocorticoid effect with no evidence of peripheral cortisol deprivation. J Clin Endocrinol Metab 1994; 78:375-80.

## Claims

1. A vaginally administrable tablet useful for treating leiomyomata, leiomyoma, myoma, uterine fibroids, endometriosis and adenomyosis, said tablet comprising mifepristone, at least one non-effervescent excipient or diluent, and at least one effervescent excipient, wherein said tablet is formulated in an immediate release form

2. A vaginally administrable tablet according to claim 1, wherein said tablet has a disintegration time of less than about 15 minutes in the vagina or between about 15 minutes and about 60 minutes in the vagina.

3. A vaginally administrable tablet according to claim 1, wherein said tablet has a disintegration time of less than about 3 minutes in water at room temperature or about 15 minutes in water at room temperature.

4. A vaginally administrable tablet according to claim 1, wherein said tablet comprises between about 0.1 to about 50 mg of mifepristone and between about 5 to about 12 wt.% effervescent excipient.

5. A vaginally administrable tablet according to claim 1, wherein said tablet comprises between about 5 to about 20 mg of mifepristone and between about 6 to 8 wt. % effervescent excipient.

6. A vaginally administrable tablet according to claim 1, wherein said tablet is configured for administration in a daily unit dosage of between about 5 mg to about 30 mg mifepristone or in a daily unit dosage of less than about 5 mg mifepristone.

7. A kit useful for treating leiomyomata, leiomyoma, myoma, uterine fibroids, endometriosis, adenomyosis and other uterine disorders, comprising:
a. a plurality of tablets;
b. an applicator for vaginal administration; and,
c. instructions for use of said tablets and said applicator;
wherein said tablet comprises mifepristone, at least one non-effervescent excipient or diluent, and at least one effervescent excipient, further wherein said tablet is formulated in an immediate release form.

8. A kit according to claim 7, wherein said tablet has a disintegration time of less than about 15 minutes in the vagina or between about 15 minutes and about 60 minutes in the vagina.

9. A kit according to claim 7, wherein said tablet has a disintegration time of less than about 3 minutes in water at room temperature or between about 3 minutes and about 15 minutes in water at room temperature.

10. A kit according to claim 7, wherein said tablet comprises between about 0.1 to about 50 mg of mifepristone and between about 5 to about 12 wt.% effervescent excipient.

11. A kit according to claim 7, wherein said tablet comprises between about 5 to about 20 mg of mifepristone.

12. A kit according to claim 7, wherein said tablet comprises between about 6 to 8 wt. % effervescent excipient.

13. A kit according to claim 7, wherein said tablet is configured for administration in a daily unit dosage of between about 5 mg to about 30 mg mifepristone or a daily unit dosage of less than about 5 mg mifepristone.

14. A tablet for vaginal administration comprising mifepristone, prepared by the steps of:
a. preparing a mixture consisting of mifepristone and at least one pharmaceutically acceptable non-effervescent excipient or diluent and at least one effervescent excipient, wherein said tablet is formulated in an immediate release form; and,
b. forming a tablet by direct compaction of said mixture said tablet.

15. A tablet for vaginal administration prepared by steps according to claim 14, wherein said mixture is further prepared by the steps of:
a. preparing a first mixture consisting of water and said mifepristone, to obtain wetted mifepristone; and, drying said wetted mifepristone to obtain dry mifepristone;
b. mixing said dry mifepristone with at least one pharmaceutically acceptable excipient or diluent to form a second mixture.

## Patentansprüche

1. Vaginal verabreichbare Tablette, zweckdienlich zur Behandlung von multiplen Leiomyomen, Leiomyom, Myom, Uterusmyomen, Endometriose und Adenomyose, wobei die Tablette Mifepriston, mindestens einen nicht brausenden Hilfsstoff oder Verschnittmittel, und mindestens einen brausenden Hilfsstoff umfasst, wobei die Tablette in Form einer Rezeptur zur sofortigen Freisetzung vorliegt.

2. Vaginal verabreichbare Tablette nach Anspruch 1, wobei die Tablette eine Zerfallszeit von weniger als ungefähr 15 Minuten in der Vagina oder zwischen ungefähr 15 Minuten und ungefähr 60 Minuten in der Vagina aufweist.

3. Vaginal verabreichbare Tablette nach Anspruch 1, wobei die Tablette eine Zerfallszeit von weniger als ungefähr 3 Minuten in Wasser auf Zimmertemperatur oder ungefähr 15 Minuten in Wasser auf Zimmertemperatur aufweist.

4. Vaginal verabreichbare Tablette nach Anspruch 1, wobei die Tablette zwischen ungefähr 0,1 bis ungefähr 50 mg Mifepriston und zwischen ungefähr 5 bis ungefähr 12 Gew.-% brausenden Hilfsstoff umfasst.

5. Vaginal verabreichbare Tablette nach Anspruch 1, wobei die Tablette zwischen ungefähr 5 bis ungefähr 20 mg Mifepriston und zwischen ungefähr 6 bis 8 Gew.-% brausenden Hilfsstoff umfasst.

6. Vaginal verabreichbare Tablette nach Anspruch 1, wobei die Tablette zur Verabreichung in einer Tages-Einzeldosis von zwischen ungefähr 5 mg bis ungefähr 30 mg Mifepriston oder in einer Tages-Einzeldosis von weniger als ungefähr 5 mg Mifepriston gestaltet ist.

7. Set zur Behandlung von multiplen Leiomyomen, Leiomyom, Myom, Uterusmyomen, Endometriose, Adenomyose und anderen Uteruserkrankungen, umfassend:
a. eine Vielzahl von Tabletten;
b. einen Applikator zur vaginalen Verabreichung; und
c. Gebrauchsanweisung für die Tabletten und den Applikator;
wobei die Tablette Mifepriston, mindestens einen nicht brausenden Hilfsstoff oder Verschnittmittel, und mindestens einen brausenden Hilfsstoff umfasst, wobei ferner die Tablette in Form einer Rezeptur zur sofortigen Freisetzung vorliegt.

8. Set nach Anspruch 7, wobei die Tablette eine Zerfallszeit von weniger als ungefähr 15 Minuten in der Vagina oder zwischen ungefähr 15 Minuten und ungefähr 60 Minuten in der Vagina aufweist.

9. Set nach Anspruch 7, wobei die Tablette eine Zerfallszeit von weniger als ungefähr 3 Minuten in Wasser auf Zimmertemperatur oder zwischen ungefähr 3 Minuten und ungefähr 15 Minuten in Wasser auf Zimmertemperatur aufweist.

10. Set nach Anspruch 7, wobei die Tablette zwischen ungefähr 0,1 bis ungefähr 50 mg Mifepriston und zwischen ungefähr 5 bis ungefähr 12 Gew.-% brausenden Hilfsstoff umfasst.

11. Set nach Anspruch 7, wobei die Tablette zwischen ungefähr 5 bis ungefähr 20 mg Mifepriston umfasst.

12. Set nach Anspruch 7, wobei die Tablette zwischen ungefähr 6 bis 8 Gew.-% brausenden Hilfsstoff umfasst.

13. Set nach Anspruch 7, wobei die Tablette zur Verabreichung in einer Tages-Einzeldosis von zwischen ungefähr 5 mg bis ungefähr 30 mg Mifepriston oder in einer Tages-Einzeldosis von weniger als ungefähr 5 mg Mifepriston gestaltet ist.

14. Tablette zur vaginalen Verabreichung, umfassend Mifepriston, hergestellt durch die Schritte des:
a. Herstellens einer Mischung, bestehend aus Mifepriston und mindestens einem pharmazeutisch akzeptablen nicht brausenden Hilfsstoff oder Verschnittmittel und mindestens einem brausenden Hilfsstoff, wobei die Tablette in Form einer Rezeptur zur sofortigen Freisetzung vorliegt; und
b. Formens einer Tablette durch direktes Kompaktieren der Mischung.

15. Tablette zur vaginalen Verabreichung, hergestellt durch Schritte nach Anspruch 14, wobei die Mischung weiter hergestellt wird durch die Schritte des:
a. Herstellens einer ersten Mischung, bestehend aus Wasser und besagtem Mifepriston, um benetztes Mifepriston zu erhalten; und Trocknens des benetzten Mifepristons, um trockenes Mifepriston zu erhalten;
b. Mischens des trockenen Mifepristons mit mindestens einem pharmazeutisch akzeptablen Hilfsstoff oder Verschnittmittel, um eine zweite Mischung zu bilden.

## Revendications

1. Comprimé apte à être administré par voie vaginale utile pour traiter une léiomatose, un léiomyome, un myome, un fibromyome utérin, une endométriose et une adénomyose, ledit comprimé comprenant de la mifépristone, au moins un diluant ou un excipient non effervescent et au moins un excipient effervescent, ledit comprimé étant formulé sous une forme à libération immédiate.

2. Comprimé apte à être administré par voie vaginale selon la revendication 1, dans lequel ledit comprimé possède un temps de désintégration inférieur à environ 15 minutes dans le vagin ou entre environ 15 minutes et environ 60 minutes dans le vagin.

3. Comprimé apte à être administré par voie vaginale selon la revendication 1, dans lequel ledit comprimé possède un temps de désintégration inférieur à environ 3 minutes dans de l'eau à la température ambiante ou d'environ 15 minutes dans de l'eau à la température ambiante.

4. Comprimé apte à être administré par voie vaginale selon la revendication 1, dans lequel ledit comprimé comprend entre environ 0,1 et environ 50 mg de mifépristone et entre environ 5 et environ 12 % en poids d'un excipient effervescent.

5. Comprimé apte à être administré par voie vaginale selon la revendication 1, dans lequel ledit comprimé comprend entre environ 5 et environ 20 mg de mifépristone et entre environ 6 et 8 % en poids d'un excipient effervescent.

6. Comprimé apte à être administré par voie vaginale selon la revendication 1, dans lequel ledit comprimé est configuré pour une administration dans une unité posologique quotidienne entre environ 5 mg et environ 30 mg de mifépristone ou dans une unité posologique quotidienne inférieure à environ 5 mg de mifépristone.

7. Nécessaire utile pour traiter une léiomatose, un léiomyome, un myome, un fibromyome utérin, une endométriose, une adénomyose et d'autres troubles utérins, comprenant :
a. plusieurs comprimés ;
b. un applicateur pour une administration par voie vaginale ; et
c. un mode d'emploi pour l'utilisation desdits comprimés et dudit applicateur ;
dans lequel ledit comprimé comprend de la mifépristone, au moins un diluant ou un excipient non effervescent et au moins un excipient effervescent, en outre dans lequel ledit comprimé est formulé sous une forme à libération immédiate.

8. Nécessaire selon la revendication 7, dans lequel ledit comprimé possède un temps de désintégration inférieur à environ 15 minutes dans le vagin ou entre environ 15 minutes et environ 60 minutes dans le vagin.

9. Nécessaire selon la revendication 7, dans lequel ledit comprimé possède un temps de désintégration inférieur à environ 3 minutes dans de l'eau à la température ambiante ou entre environ 3 minutes et 15 minutes dans de l'eau à la température ambiante.

10. Nécessaire selon la revendication 7, dans lequel ledit comprimé comprend entre environ 0,1 et environ 50 mg de mifépristone et entre environ 5 et environ 12 % en poids d'un excipient effervescent.

11. Nécessaire selon la revendication 7, dans lequel ledit comprimé comprend entre environ 5 et environ 20 mg de mifépristone.

12. Nécessaire selon la revendication 7, dans lequel ledit comprimé comprend entre environ 6 et 8 % en poids d'un excipient effervescent.

13. Nécessaire selon la revendication 7, dans lequel ledit comprimé est configuré pour une administration dans une unité posologique quotidienne entre environ 5 mg et environ 30 mg de mifépristone ou dans une unité posologique quotidienne inférieure à environ 5 mg de mifépristone.

14. Comprimé pour une administration par voie vaginale, comprenant de la mifépristone, que l'on prépare en passant par les étapes consistant à :
a. préparer un mélange constitué par de la mifépristone et par au moins un diluant ou un excipient non effervescent pharmaceutiquement acceptable et au moins un excipient effervescent, ledit comprimé étant formulé sous une forme à libération immédiate ; et
b. former un comprimé par compression directe dudit mélange.

15. Comprimé pour une administration par voie vaginale préparé en passant par les étapes selon la revendication 14, ledit mélange étant en outre préparé en passant par les étapes consistant à :
a. préparer un premier mélange constitué par de l'eau et ladite mifépristone, afin d'obtenir de la mifépristone humidifiée et sécher ladite mifépristone humidifiée pour obtenir de la mifépristone sèche ;
b. mélanger ladite mifépristone sèche avec au moins un diluant ou un excipient pharmaceutiquement acceptable pour obtenir un deuxième mélange.
